# EUROPEAN PATENT SPECIFICATION

(11) **EP 0 746 986 B1**
(45) Date of publication and mention of the grant of the patent: **23.08.2000**
(21) Application number: 96108849.9
(22) Date of filing: 03.06.1996
(51) Int. Cl.: A23L 1/22, A24B 15/30, A24B 15/34

(54) **Delayed release flavourant compositions**
Aromazusammensetzungen mit verzögerter Freigabe
Compositions aromatiques à libération prolongée

(30) Priority: 08.06.1995 EP 95810375
(43) Date of publication of application: 11.12.1996
(73) Proprietor: GIVAUDAN-ROURE (INTERNATIONAL) S.A., 1214 Vernier, Genève (CH)
(72) Inventor: Anderson, Denise, 8032 Zurich (CH)
(74) Representative: Buntz, Gerhard

(56) References cited:
- EP-A- 0 578 421
- US-A- 5 320 131
- PATENT ABSTRACTS OF JAPAN vol. 013, no. 388 (C-630), 28 August 1989 & JP 01 135867 A (ADEKA ARGUS CHEM CO LTD)

## Description

The invention concerns delayed release flavourant compositions.

In particular, these compositions are selected from the group consisting of
a) smoking compositions and
b) foodstuffs, in particular
b1) bakeable foodstuffs and
b2) microwaveable foodstuffs
containing flavour-release additives, namely 1,2-diols of the formula 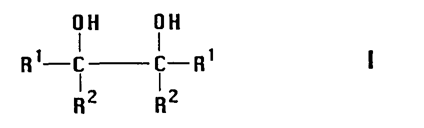 wherein R¹ is one of the radicals 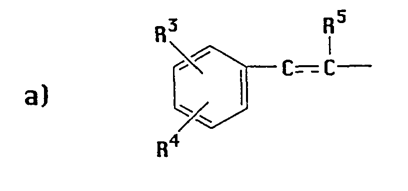 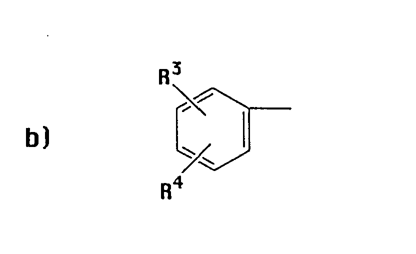 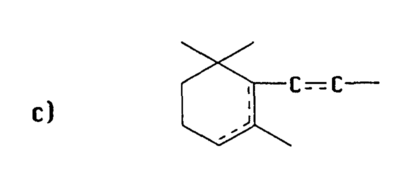 where
- R² is: H, lower alkyl
- R³, R⁴ are: H, lower alkyl, lower alkoxy, hydroxy, or R³ + R⁴ together are methylenedioxy,
- R⁵ is: lower alkyl
and the broken lines represent in the ring one additional bond and in the side chain an optional bond.

These diols I possess no detectable flavour or odour themselves at normal temperatures and atmospheric conditions but function mainly as flavour precursors, in as far as they release a flavourant upon heating at higher temperatures, e.g. temperatures leading to pyrolysis and thermolysis respectively. The diols I thus may be used as flavourants in smoking compositions, e.g. in tobacco compositions or tobacco substitutes, as sustained flavourants and odorants to mask or enhance the flavours and odours of burning tobacco products, as flavour additives to foods, in particular bakeable and microwaveable foods.

EP 578 421 A, describes smoking compositions which contain a beta -hydroxy-carboxylate flavorant-release additives for smoking compositions used at 0.01-5 wt.% of the paper wrapper (pref. in the side-seam adhesive). On pyrolysis, the flavorant release additives afford volatile flavourants.

US 5 320 131 A, describes a cigarette having a circumscribing wrapping material containing an aromatic and flavourful precursor formed by contacting 2,3-pinanediol with trans-cinnamaldehyde.

The term lower alkyl and the alkyl portion of lower alkoxy radical suitably refers to straight or branched alkyl residues carrying from 1 to 6 carbon atoms. Suitable examples for the individual residues are as follows:
- R²: H, CH₃, C₂H₅, etc.
- R³, R⁴: H, CH₃, C₂H₅, n- or iso- C₃H₇, t-C₄H₉, etc.
- R⁵: CH₃, C₂H₅, n- or iso- C₃H₇, t-C₄H₉, C₅H₁₁, C₆H₁₃, etc.

Although the two radicals R¹ of formula I may be different from each other and the two radicals R² may also be diffent from each other, in the preferred aspects these radicals R¹ and R² respectively are the same.

As used herein the term "organoleptic" refers to compounds I which stimulate the sense of smell or taste, and are thus perceived as having a characteristic odour and/or flavour.

The terms "odour", "fragrance" and "smell" are used interchangeably whenever a compound is referred to as an organoleptic which is intended to stimulate the sense of smell.

The terms "flavour", "flavouring" and "flavourant" are also used interchangeably whenever an organoleptic compound is referred to which is intended to stimulate the sense of taste.

An "organoleptically effective amount" is a level or amount of the compounds(s) I present in a material at which the incorporated compound(s) exhibit(s) a sensory effect, e.g. by stimulating the sense of smell or taste.

The terms "tobacco" and "tobacco substitutes" are used in the conventional sense and include smokable as well as non-smokable forms in which tobacco is regularly used, e.g. cigarettes, snuff chewable compositions, etc.

The term "tobacco paper" refers to smokable paper used to contain tobacco, e.g. tobacco rolling paper.

The terms "food" and "foodstuff' are used interchangeably.

The term "food product" would consequently designate the product of the process to which the foodstuff(s) containing the diol I is (are) subjected to.

The term "tobacco substitute" is meant to include non-tobacco filled materials such as smoking products from sugar beet pulp, coffee bean hulls and other cellulosic or carbohydrate materials, etc.

The preferred compounds I are
1,6-diphenyl-1,5-hexadiene-3,4-diol,
2,5-dihexyl-1,6-diphenyl-1,5-hexadiene-3,4-diol and
1,2-bis(4-hydroxy-3-methoxyphenyl)-1,2-ethanediol.

Further suitable compounds I include
3,4-dimethyl-1,6-bis(2,6,6-trimethyl-1-cyclohexen-1-yl)-1,5-hexadiene-3,4-diol,
3,4-dimethyl-1,6-bis(2,6,6-trimethyl-2-cyclohexen-1-yl)-1,5-hexadiene-3,4-diol,
1,2-bis(1,3-benzodioxol-5-yl)-1,2-ethanediol,
1,2-bis(4-methoxyphenyl)-1,2-ethanediol,
1,2-diphenyl-1,2-ethanediol or
1,6-bis(4-methoxyphenyl)-1,5-hexadiene-3,4-diol.

The compounds I possess organoleptic properties and therefore permit the development of methods useful in enhancing the flavour of foods. These compounds are also useful in enhancing the odour, masking any unpleasant odour or enhancing the flavour of tobacco products.

These compounds may be used individually in an amount effective to enhance a characteristic flavour or odour of a given material. The compounds may also be mixed with other flavour or fragrance components in an amount sufficient to provide the desired flavour or odour characteristic.

The amount required to produce the desired, overall effect varies depending upon the particular compound chosen, the product in which it will be used, and the particular effect desired.

For example, depending upon the selction and concentration of the compounds I used, addition of the compounds I either singly or as a mixture to cigarette tobacco at levels ranging from about 5 ppm to about 50 000 ppm tends to enhance the smoking flavour and/or mask undesirable smoking odours. An important property of these compounds I is that the flavourant or odorant is covalently bound as an non-volatile compound and it is only when the tobacco product is ignited and burns that the flavourant or odorant is released.

Addition of the diols I of formula I either separately or as a mixture at levels suitably ranging from about 5 ppm to about 50 000 ppm by weight onto the media enclosing the tobacco serves to incorporate the odorant/flavourant in the side-stream smoke as the tobacco product burns. Air borne flavourants and/or odorants along with other combustion products are thus introduced. This newly formed odorant or flavourant serves to enhance or mask the smoking odours depending upon selection and use levels of the compounds I.

The diols I are also particularly useful in the flavouring and aromatizing of cooked foods. Addition of the diols either singly or as a mixture to a cake batter, e.g. a microwave cake batter, serves to impart appropriate baking aromas to the cake as it is heated in the microwave as well as impart flavouring in the finished product. Typically, diols I are employed at levels ranging from about 0.0005 to about 10%, in particular from 0.05 to about 5.00%.

In addition to the baking aromas, other pleasant organoleptic properties may be provided, the palette is broad and may comprise - depending on the compound I used - floral, fruity, sweet, herbaceous, balsamic, spicy, cinnamon, woody, vanillin notes.

Notewortly are, furthermore, the stability of the novel flavourants.

A suitable temperature range may extend from about 70°C to about 300°C, or even more.

The compounds I may be incorporated into the foodstuff or the smoking composition, e.g. the tobacco product along with other ingredients. Such other ingredients include emulsifiers, carriers, binders, sweeteners, stabilizers, buffers and solvents, etc.

The addition of the diol I may generally be effected according to methods known in the art, e.g. as such or dissolved in any suitable solvent, e.g. in an alcohol, such as ethanol or aqueous ethanol, etc. It may then be sprayed or injected into the tobacco and/or tobacco substitute matrix. Such method ensures an even distribution of the flavourant additive throughout the filler, and thereby facilitates the production of a more uniform smoking composition. Alternatively, the flavourant may be incorporated as part of a concentrated tobacco extract which is applied to a fibrous tobacco web as in the manufacture of reconstituted tobacco. Another suitable procedure is to incorporate the flavourant in tobacco or tobacco substitute filler in a concentration between about 0.5-5 weight percent, based on the weight of filler, and then subsequently to blend the treated filler with filler which does not contain flavourant additive. Naturally, the additive can also be present as a surface coating or absorbed component of the paper wrapper, and/or the additive can be incorporated as a component of the adhesive formulation which is utilized to seal the sideseam of cigarette paper wrappers.

A large number of the compounds I - actually the majority thereof are known. In as far as the novel compounds are concerned, the following applies:

Pinacol diols can be prepared from the corresponding aldehydes and ketones by standard methods known to those skilled in the art - see Comprehensive Organic Synthesis, Barry M. Trost, ed., Vol. 3, 1991, pp 563-579. For example, reaction of aldehydes or ketones with metals - e.g. Zn, Mg, Ce, Al(Hg) or Ti - and acid - e.g. acetic acid - provides pinacol diols I. Various aldehydes and ketones can also be transformed by electrochemical means to their pinacol diols. Pinacol diols can also be prepared by reduction of benzoins. All these methods are outlined by B.M. Trost.

The two novel compounds of this invention, i.e. 1,6-bis (4-methoxyphenyl)-1,5-hexadiene-3,4-diol and 2,5-dihexyl-1,6-diphenyl-1,5-hexadiene-3,4-diol are thus easily obtained by subjecting 1,4-methoxycinnamaldehyde or α-hexylcinnamaldehyde to pinacolisation as outlined above.

The following examples are set forth herein to illustrate methods of synthesis of the pinacol diol derivatives I and their use as flavourants and flavour precursors. These examples are intended only to illustrate the embodiments of this invention and are in no way meant to limit the scope thereof.

All parts, proportions, percentages and ratios used herein are by weight unless otherwise indicated.

### Example 1

### 1,6-Diphenyl-1,5-hexadiene-3,4-diol

A mixture of cinnamaldehyde (200 g, 1.5 mol), zinc (98.9 g, 1.5 mol), water (380 ml), and ether (230 ml) was heated to reflux under a nitrogen atmosphere. Acetic acid (175 ml) was added over a 2 hour period. The reaction was heated at reflux for an additional 3 hours. The mixture was cooled and filtered. Ethyl acetate and ether (1:2) were added and the mixture was washed sequentially with water (2 x), aqueous sodium bicarbonate solution until neutral, brine, and dried over sodium sulfate, filtered, and the solvent was evaporated under reduced pressure. The syrup obtained was crystallized from ethyl acetate to give meso-1,6-diphenyl-1,5-hexadiene-3,4-diol.
mp: 150-152°C, ¹H-NMR (CDCl₃) δ 7.42-7.20 (10 H, m), 6.71 (2 H, d, J = 15.87 Hz), 6.29 (2 H, dd, J = 6.41 and 15.87 Hz), 4.49-4.39 (2 H, m), 2.24 (2 H, OH). IR (KBr) 3298, 2908, 1449, 963, 746 cm⁻¹. MS m/e (% abundance) 266 (1), 133 (100), 115 (22), 77 (25), 55 (67).
d,l-1,6-Diphenyl-1,5-hexadiene-3,4-diol can be isolated from the reaction mixture.
¹H-NMR (CDCl₃) δ 7.42-7.20 (10 H, m), 6.72 (2 H, d, J = 16.17 Hz), 6.34-6.18 (2 H, m), 4.32-4.24 (2 H, m), 2.55 (2 H, s). IR (KBr) 3313, 1449, 1047, 970, 690 cm⁻¹. MS m/e (% abundance) 266 (1), 133 (100), 115 (25), 55 (29).

### Example 2

### 1,6 -Bis(4-methoxyphenyl)-1,5-hexadiene-3,4-diol

In a fashion similar to that described in Example 1, 1,4-methoxycinnamaldehyde was treated with zinc and acetic acid to provide 1,6-bis(4-methoxyphenyl)-1,5-hexadiene-3,4-diol.
mp: 145-146°C. ¹H-NMR (CDCl₃) δ7.34-7.28 (4 H, m), 6.86-6.61 (4 H, m), 6.69-6.59 (2 H, m), 6.29-6.06 (2 H, m), 3.80 (6 H, s). IR (KBr) 3365, 2956, 2838, 1606, 1512, 1252, 1031 cm⁻¹. MS m/e (% abundance) 326 (1), 163 (100), 55 (18).

### Example 3

### 2,5-Dihexyl-1,6-diphenyl-1,5-hexadiene-3,4-diol

In a fashion similar to that described in Example 1, α-hexylcinnamaldehyde was treated with zinc and acetic acid to provide 2,5-dihexyl-1,6-diphenyl-1,5-hexadiene-3,4-diol.
mp: 86-86.5, ¹H-NMR (CDCl₃) δ 7.38-7.16 (10 H, m), 6.73 (2 H, bs), 4.33 (2 H, bs), 2.56-2.46 (4 H, m), 2.14-2.06 (2 H, m), 1.60-1.49 (4 H, m), 1.40-1.21 (10 H, m), 0.90-0.84 (6 H, m). IR (KBr) 3455, 3296, 2922, 2853, 1454, 1091, 699 cm⁻¹. MS m/e (% abundance) 434 (3), 218 (46), 217 (100), 91 (22).

### Example 4

### Preparation of an α-hexylcinnamaldehyde cigarette

An ethyl acetate solution of the compound from Example 3 was applied to cigarette papers at the rate of 1000 ppm. (Application rates of 5 to 50'000 ppm may actually be useful). The paper was incorporated into cigarettes. Prior to smoking, no odour of a-hexylcinnamaldehyde was observed. Upon smoking, a noticeable and persistent floral odour was observed in the room air. The floral odour was, actually, already observed on first puff.

### Example 5

### Preparation of a vanillin cigarette

An alcohol/water solution of the compound 1,2-bis(4-hydroxy-3-methoxyphenyl)-1,2-ethanediol was applied to cigarette papers at the rate of 500 ppm. The paper was incorporated into cigarettes. Prior to smoking, no odor of vanillin was observed. Upon smoking a faint, but distinct odour of vanillin was observed in the room air.

## Claims

1. A delayed release flavourant composition, selected from the group consisting of a) a smoking composition comprising an admixture of a combustible filler being natural tobacco, reconstituted tobacco or a tobacco substitute, and b) a foodstuff containing an organoleptically effective amount of a flavorant-release additive, the flavourant-release additive being a 1,2 diol of the formula 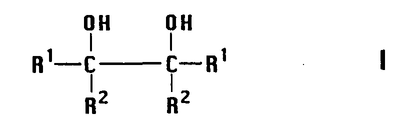 wherein R¹ is one of the radicals 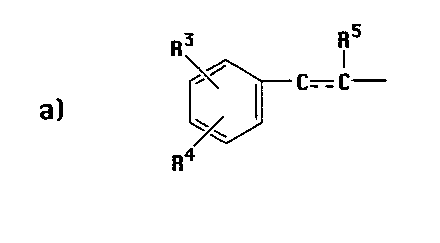 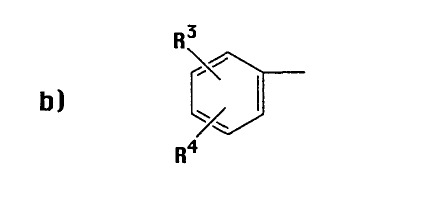 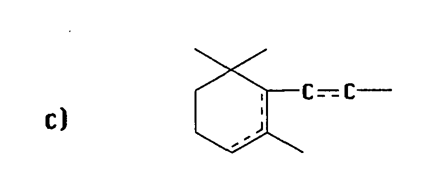 where
R² is H, lower alkyl
R³, R⁴ are H, lower alkyl, lower alkoxy, hydroxy, or R³ + R⁴ together are methylenedioxy,
R⁵ is lower alkyl
and the broken lines represent in the ring one additional bond and in the side chain an optional bond.

2. A smoking composition of claim 1 which is a cigarette smoking product comprising a combustible filler being natural tobacco, reconstituted tobacco or a tobacco substitute, and a paper wrapper which has incorporated therein a flavorant-release additive, the flavorant-release additive being a 1,2 diol of the formula 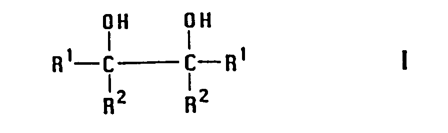 wherein R¹ is one of the radicals 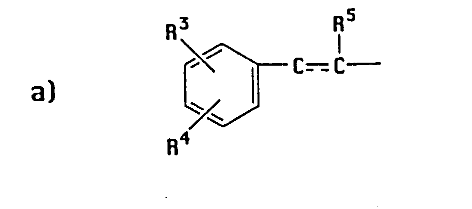 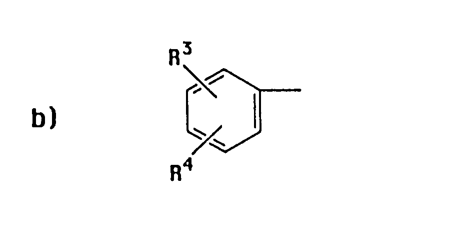 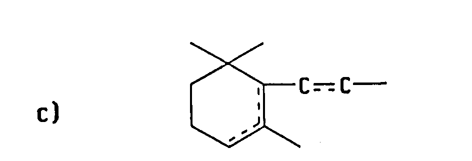 where
R² is H, lower alkyl
R³, R⁴ are H, lower alkyl, lower alkoxy, hydroxy, or R³ + R⁴ together are methylenedioxy,
R⁵ is lower alkyl
and the broken lines represent in the ring one additional bond and in the side chain an optional bond.

3. A composition according to Claim 1, wherein the foodstuff is a bakeable foodstuff.

4. A composition according to Claim 1, wherein the foodstuff is a microwaveable foodstuff.

5. A composition according to any one of Claims 1 to 4, wherein the concentration of the flavourant - release additive is ca. 0,0005 to ca. 10 % , preferably ca.0,005 to ca. 5 % w/w.

6. A composition according to any one of Claims 1 to 5, wherein the flavourant additive is released preferably during ignition and burning of the smoking composition, e.g. the tobacco product or during the baking or the microwaving process of the foodstuff.

7. A composition according to any one of Claims 1 to 6, wherin R³ and R⁴ in the formula I a) diol is hydrogen.

8. A composition according to any one of Claims 1 to 6, wherein R² is hydrogen

9. A composition according to any one of Claims 1 to 6, wherein R² in the formula I b) diol is methyl.

10. A composition according to any one of Claims 1 to 6, wherein R² in the formula I c) diol is methyl and there is an optional bond in the side chain of the molecule.

11. A composition according to any one of Claims 1 to 6, wherein the compound of Formula I is
1,6-diphenyl-1,5-hexadiene-3,4-diol,
3,4-dimethyl-1,6-bis(2,6,6-trimethyl-1-cyclohexen-1-yl)-1,5-hexadiene-3,4-diol,
3,4-dimethyl-1,6-bis(2,6,6-trimethyl-2-cyclohexen-1-yl)-1,5-hexadiene-3,4-diol,
1,2-bis(4-hydroxy-3-methoxyphenyl)-1,2-ethanediol,
1,2-bis(1,3-benzodioxol-5-yl)-1,2-ethanediol,
1,2-bis(4-methoxyphenyl)-1,2-ethanediol,
1,2-diphenyl-1,2-ethanediol,
1,6-bis(4-methoxyphenyl)-1,5-hexadiene-3,4-diol or
2,5-dihexyl-1,6-diphenyl-1,5-hexadiene-3,4-diol.

12. A method of flavouring a smoking composition, e.g. tobacco, or a food, comprising adding to the smoking composition, e.g. tobacco, or treating a food with an organoleptically effective amount of a 1,2 - diol of the formula 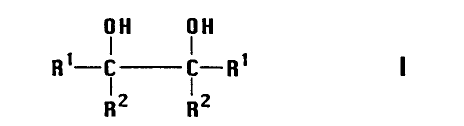 wherein R¹ is one of the radicals 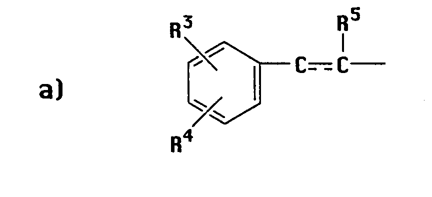 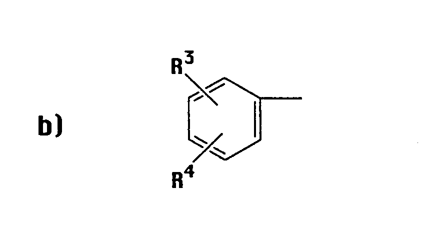 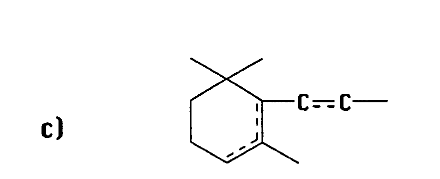 where
R² is H, lower alkyl
R³, R⁴ are H, lower alkyl, lower alkoxy, hydroxy, or R³ + R⁴ together are methylenedioxy,
R⁵ is lower alkyl
and the broken lines represent in the ring one additional bond and in the side chain an optional bond.

13. A method of flavouring a smoking composition, e.g. tobacco, or a food, comprising igniting and burning the smoking composition, e.g. tobacco, or baking or microwaving the foodstuff containing an organoleptically effective amount of a 1,2 -diol of the formula 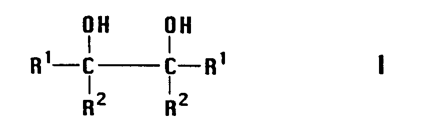 wherein R¹ is one of the radicals 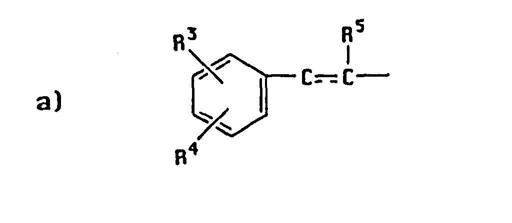 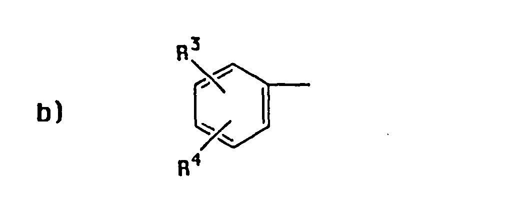 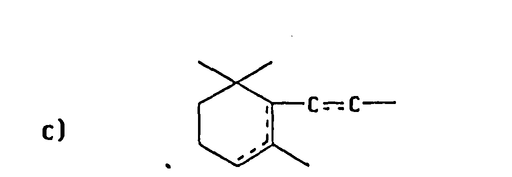 where
R² is H, lower alkyl
R³, R⁴ are H, lower alkyl, lower alkoxy, hydroxy, or R³ + R⁴ together are methylenedioxy,
R⁵ is lower alkyl
and the broken lines represent in the ring one additional bond and in the side chain an optional bond.

14. A method according to Claim 12 or 13, wherein the 1,2 diol of Formula I is
1,6-diphenyl-1,5-hexadiene-3,4-diol,
3,4-dimethyl-1,6-bis(2,6,6-trimethyl-1-cyclohexen-1-yl)-1,5-hexadiene-3,4-diol,
3,4-dimethyl-1,6-bis(2,6,6-trimethyl-2-cyclohexen-1-yl)-1,5-hexadiene-3,4-diol,
1,2-bis(4-hydroxy-3-methoxyphenyl)-1,2-ethanediol,
1,2-bis(1,3-benzodioxol-5-yl)-1,2-ethanediol,
1,2-bis(4-methoxyphenyl)-1,2-ethanediol,
1,2-diphenyl-1,2-ethanediol,
1,6-bis(4-methoxyphenyl)-1,5-hexadiene-3,4-diol or
2,5-dihexyl-1,6-diphenyl-1,5-hexadiene-3,4-diol.

15. 1,6-bis (4-Methoxyphenyl)-1,5-hexadiene-3,4-diol.

16. 2,5-Dihexyl-1,6-diphenyl-1,5-hexadiene-3,4-diol.

17. Process for the preparation of 1,6-bis (4-methoxyphenyl)-1,5-hexadiene-3,4-diol or of 2,5-dihexyl-1,6-diphenyl-1,5-hexadiene-3,4-diol, comprising subjecting 1,4 -methoxycinnamaldehyde or α-hexylcinnamaldehyde to pinacolisation in a manner known per se.

18. The use of the 1,2 - diols of the formula 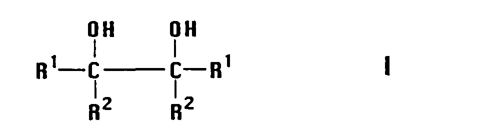 wherein R¹ is one of the radicals 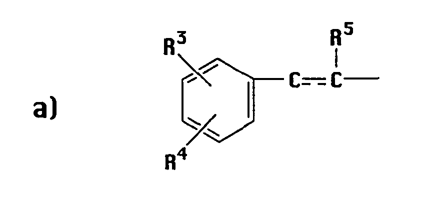 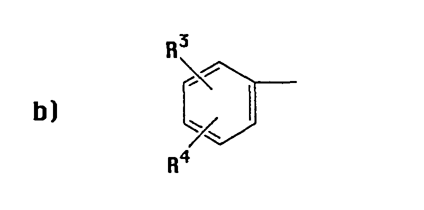 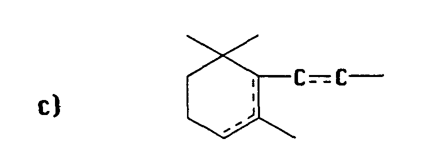 where
R² is H, lower alkyl
R³, R⁴ are H, lower alkyl, lower alkoxy, hydroxy, or R³ + R⁴ together are methylenedioxy,
R⁵ is lower alkyl
and the broken lines represent in the ring one additional bond
and in the side chain an optional bond,
as delayed flavourant release additives for smoking compositions and for food.

## Patentansprüche

1. Aromazusammensetzung mit verzögerter Freigabe ausgewählt aus der Gruppe bestehend aus
a) einer Rauchzusammensetzung umfassend eine Mischung aus einem verbrennbaren Füllstoff, der natürlicher Tabak, rekonstituierter Tabak oder ein Tabakersatzstoff ist und
b) einem Lebensmittel, das eine organoleptisch wirksame Menge eines ein Aroma freisetzenden Additivs enthält, wobei das Aroma freisetzende Additiv ein 1,2-Diol der Formel 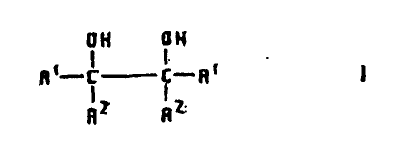 ist, worin
R¹ einer der Reste 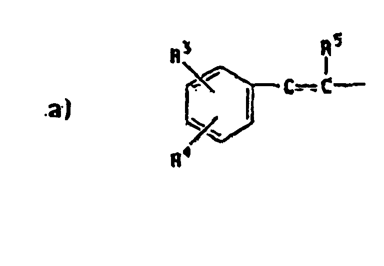 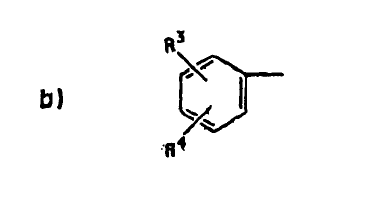 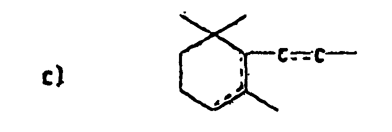 ist, worin
R² H oder ein Niedrigalkylrest ist,
R³, R⁴ H, Niedrigalkylreste, Niedrigalkoxyreste, Hydroxyreste sind oder R³ und R⁴ zusammen einen Methylendioxyrest bilden,
R⁵ ein Niedrigalkylrest ist und
die durchbrochenen Linien im Ring eine zusätzliche Bindung darstellen und in der Seitenkette eine fakultative Bindung darstellen.

2. Rauchzusammensetzung nach Anspruch 1, die ein Zigarettenrauchprodukt ist mit einem verbrennbaren Füllstoff, der natürlicher Tabak, rekonstituierter Tabak oder ein Tabakersatzstoff ist, und einer Papierumhüllung, in der ein Aroma freisetzendes Additiv enthalten ist, wobei das Aroma freisetzende Additiv ein 1,2-Diol der Formel 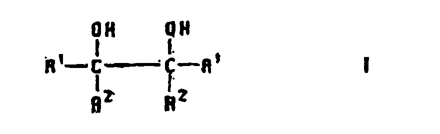
ist, worin R¹ einer der Reste 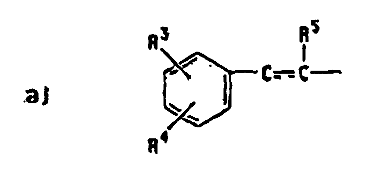 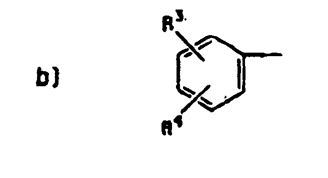 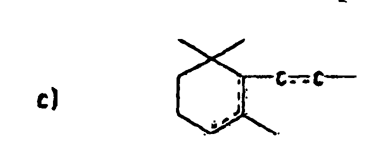 ist, worin
R² H oder ein Niedrigalkylrest ist,
R³, R⁴ H, Niedrigalkylreste, Niedrigalkoxyreste, Hydroxyreste sind oder R³ und R⁴ zusammen einen Methylendioxyrest bilden,
R⁵ ein Niedrigalkylrest ist und
die durchbrochenen Linien im Ring eine zusätzliche Bindung darstellen und in der Seitenkette eine fakultative Bindung darstellen.

3. Zusammensetzung nach Anspruch 1, worin das Lebensmittel ein backfähiges Lebensmittel ist.

4. Zusammensetzung nach Anspruch 1, worin das Lebensmittel ein mit Mikrowellen zu behandelndes Lebensmittel ist.

5. Zusammensetzung nach einem der Ansprüche 1 bis 4, worin die Konzentration des Aroma freisetzenden Additivs ca. 0,0005 bis ca. 10%, bevorzugt 0,005 bis ca. 5% G/G ist.

6. Zusammensetzung nach einem der Ansprüche 1 bis 5, worin das Aromaadditiv bevorzugt während des Anzündens und Verbrennens der Rauchzusammensetzung, z.B. des Tabakprodukts, oder während des Backens oder der Mikrowellenbehandlung des Lebensmittels freigesetzt wird.

7. Zusammensetzung nach einem der Ansprüche 1 bis 6, worin R³ und R⁴ in dem Diol der Formel Ia) Wasserstoff sind.

8. Zusammensetzung nach einem der Ansprüche 1 bis 6, worin R² Wasserstoff ist.

9. Zusammensetzung nach einem der Ansprüche 1 bis 6, worin R² in dem Diol der Formel Ib) ein Methylrest ist.

10. Zusammensetzung nach einem der Ansprüche 1 bis 6, worin R² in dem Diol der Formel Ic) ein Methylrest ist und in der Seitenkette des Moleküls eine fakultative Bindung vorliegt.

11. Zusammensetzung nach einem der Ansprüche 1 bis 6, worin die Verbindung der Formel I
1,6-Diphenyl-1,5-hexadien-3,4-diol,
3,4-Dimethyl-1,6-bis(2,6,6-trimethyl-l-cyclohexen-1-yl)-1,5-hexadien-3, 4-diol,
3, 4-Dimethyl-1,6-bis (2, 6, 6-trimethyl-2-cyclohexen-1-yl)-1,5-hexadien-3,4-diol,
1,2-Bis(4-hydroxy-3-methoxyphenyl)-1,2-ethandiol,
1,2-Bis (1,3-benzodioxol-5-yl)-1,2-ethandiol,
1,2-Bis (4-methoxyphenyl)-1,2-ethandiol,
1,2-Diphenyl-1,2-ethandiol,
1,6-Bis (4-methoxyphenyl)-1,5-hexadien-3,4-diol oder
2, 5-Dihexyl-1,6-diphenyl-1,5-hexadien-3,4-diol
ist.

12. Verfahren zur Aromatisierung einer Rauchzusammensetzung, z.B. von Tabak, oder eines Lebensmittels, umfassend, dass man der Rauchzusammensetzung, z.B. dem Tabak eine organoleptisch wirksame Menge eines 1,2-Diols der Formel 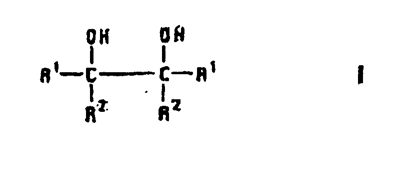
worin R¹ einer der Reste 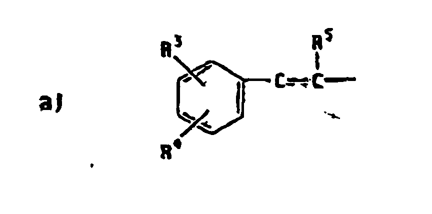 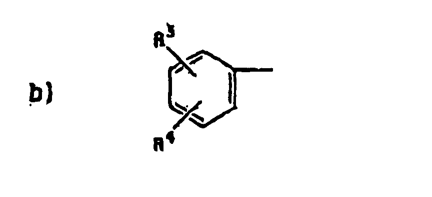 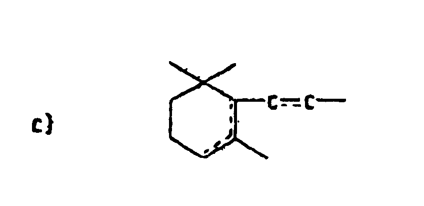 ist, worin
R² H oder ein Niedrigalkylrest ist,
R³, R⁴ H, Niedrigalkylreste, Niedrigalkoxyreste, Hydroxyreste sind oder R³ und R⁴ zusammen einen Methylendioxyrest bilden,
R⁵ ein Niedrigalkylrest ist und
die durchbrochenen Linien im Ring eine zusätzliche Bindung darstellen und in der Seitenkette eine fakultative Bindung darstellen, zugibt oder ein Lebensmittel mit einer organoleptisch wirksamen Menge des 1,2-Diols behandelt.

13. Verfahren zur Aromatisierung einer Rauchzusammensetzung, z.B. Tabak, oder eines Lebensmittels, umfassend, dass man die Rauchzusammensetzung, z.B. Tabak, anzündet und verbrennt, oder das Lebensmittel backt oder mit Mikrowellen behandelt, wobei die Zusammensetzung eine organoleptisch wirksame Menge eines 1,2-Diols der Formel 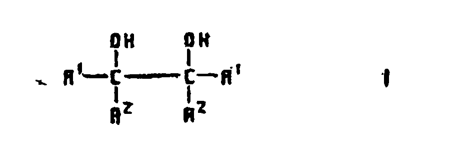 enthält, worin
R¹ einer der Reste 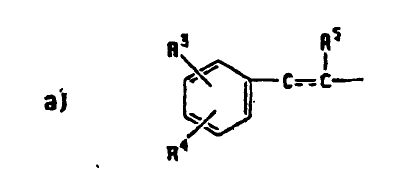 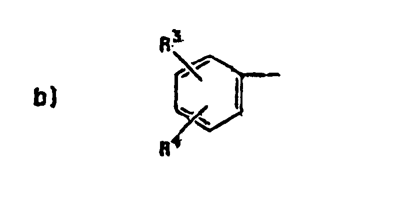 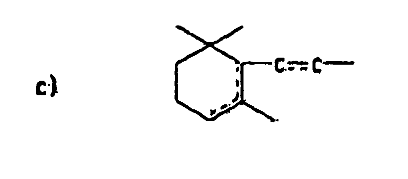 ist, worin
R² H oder ein Niedrigalkylrest ist,
R³, R⁴ H, Niedrigalkylreste, Niedrigalkoxyreste, Hydroxyreste sind oder R³ und R⁴ zusammen einen Methylendioxyrest bilden,
R⁵ ein Niedrigalkylrest ist und

14. Verfahren nach einem der Ansprüche 12 oder 13, worin das
1,2-Diol der Formel I
1,6-Diphenyl-1,5-hexadien-3,4-diol,
3,4-Dimethyl-1,6-bis(2,6,6-trimethyl-1-cyclohexen-1-yl)1,5-hexadien-3,4-diol,
3,4-Dimethyl-1,6-bis(2,6,6-trimethyl-2-cyclohexen-1-yl)1,5-hexadien-3,4-diol,
1,2-Bis (4-hydroxy-3-methoxyphenyl)-1,2-ethandiol,
1,2-Bis(1,3-benzodioxol-5-yl)-1,2-ethandiol,
1,2-Bis(4-methoxyphenyl)-1,2-ethandiol,
1,2-Diphenyl-1,2-ethandiol,
1,6-Bis(4-methoxyphenyl)-1,5-hexadien-3,4-diol oder
2,5-Dihexyl-1,6-diphenyl-1,5-hexadien-3,4-diol
ist.

15. 1,6-Bis (4-methoxyphenyl)-1,5-hexadien-3,4-diol.

16. 2,5-Dihexyl-1,6-diphenyl-1,5-hexadien-3,4-diol.

17. Verfahren zur Herstellung von 1,6-Bis(4-methoxyphenyl)1,5-hexadien-3,4-diol oder 2, 5-Dihexyl-1,6-diphenyl-1,5-hexadien-3,4-diol, umfassend, dass man 1,4-Methoxyzimtaldehyd oder α-Hexylzimtaldehyd einer Pinacolisierung in an sich bekannter Weise unterzieht.

18. Verwendung von 1,2-Diolen der Formel 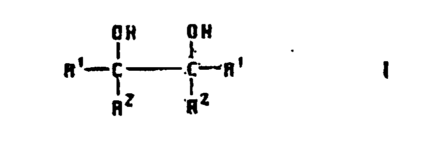
worin R¹ einer der Reste 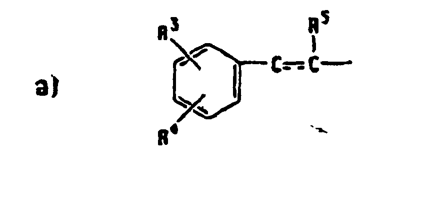 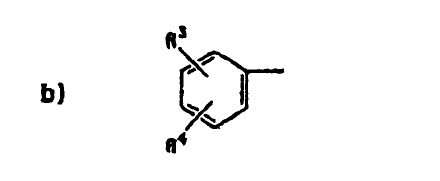 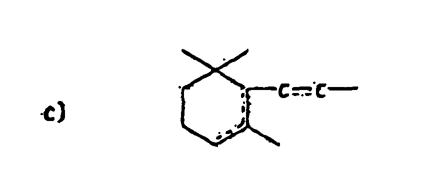 ist, worin
R² H oder ein Niedrigalkylrest ist,
R³, R⁴ H, Niedrigalkylreste, Niedrigalkoxyreste, Hydroxyreste sind oder R³ und R⁴ zusammen einen Methylendioxyrest bilden,
R⁵ ein Niedrigalkylrest ist und
die durchbrochenen Linien im Ring eine zusätzliche Bindung darstellen und in der Seitenkette eine fakultative Bindung darstellen,
als verzögert Aroma freisetzende Additive für Rauchzusammensetzungen und für Lebensmittel.

## Revendications

1. Composition aromatique à libération prolongée, choisie dans le groupe constitué par a) une composition à fumer comprenant un mélange d'une charge combustible qui est du tabac naturel, du tabac reconstitué ou un succédané de tabac, et b) un aliment contenant une quantité organoleptiquement efficace d'un additif libérant un arôme, l'additif libérant un arôme étant un 1,2-diol de formule 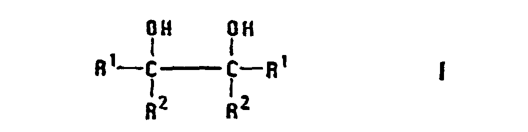
dans laquelle R¹ est l'un des radicaux 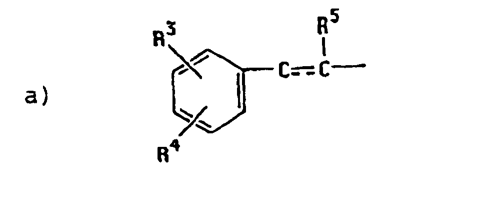 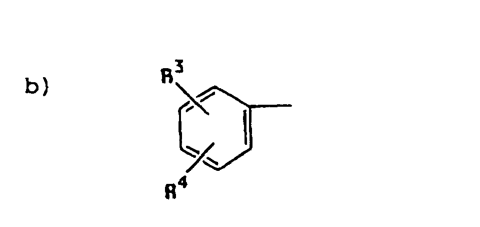 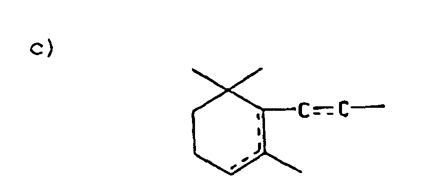
où R² représente H ou un alkyle inférieur,
R³ et R⁴ représentent chacun H, un alkyle inférieur, un alcoxy inférieur, un hydroxy, ou
R³ et R⁴ représentent ensemble un méthylenedioxy,
R⁵ est un alkyle inférieur,
et les lignes pointillées représentent dans le noyau une liaison supplémentaire et dans la chaîne latérale une liaison facultative.

2. Composition à fumer de la revendication 1 qui est un produit à fumer de type cigarette comprenant une charge combustible, laquelle est du tabac naturel, du tabac reconstitué ou un succédané de tabac, et une enveloppe en papier dans laquelle est incorporé un additif libérant un arôme, l'additif libérant un arôme étant un 1,2-diol de formule 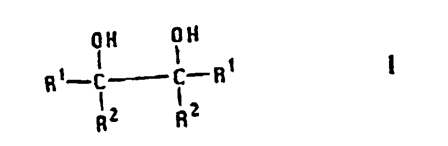
dans laquelle R¹ est l'un des radicaux 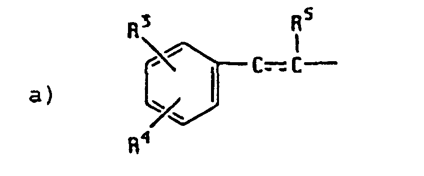 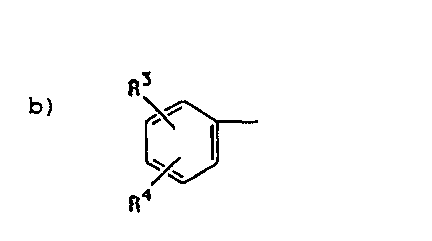 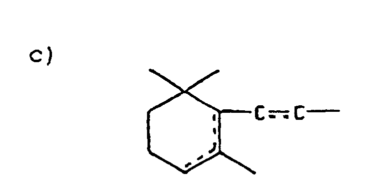
où R² représente H ou un alkyle inférieur,
R³ et R⁴ représentent chacun H, un alkyl inférieur, un alcoxy inférieur, un hydroxy, ou
R³ et R4 ensemble représentent un méthylènedioxy,
R⁵ est un alkyle inférieur,
et les lignes pointillées représentent dans le noyau une liaison supplémentaire et dans la chaîne latérale une liaison facultative.

3. Composition selon la revendication 1, dans laquelle l'aliment est un aliment à cuire.

4. Composition selon la revendication 1, dans laquelle l'aliment est un aliment susceptible d'être cuit au four à micro-ondes.

5. Composition selon l'une quelconque des revendications 1 à 4, dans laquelle la concentration de l'additif libérant un arôme est d'environ 0,0005 à environ 10%, de préférence d'environ 0,005 à environ 5% p/p.

6. Composition selon l'une quelconque des revendications 1 à 5, dans laquelle l'additif aromatisant est libéré de préférence au cours de l'allumage et de la combustion de la composition à fumer, par exemple le produit de tabac, ou au cours de la cuisson ou du passage au four à micro-ondes de l'aliment.

7. Composition selon l'une quelconque des revendications 1 à 6, dans laquelle R³ et R⁴ dans le diol de formule Ia) représentent l'hydrogène.

8. Composition selon l'une quelconque des revendications 1 à 6, dans laquelle R² représente l'hydrogène.

9. Composition selon l'une quelconque des revendications 1 à 6, dans laquelle R² dans le diol de formule Ib) est le méthyle.

10. Composition selon l'une quelconque des revendications 1 à 6, dans laquelle R² dans le diol de formule Ic) est un méthyle et il y a une liaison facultative dans la chaîne latérale de la molécule.

11. Composition selon l'une quelconque des revendications 1 à 6, dans laquelle le composé de formule I est le
1, 6-diphényl-1,5-hexadiène-3,4-diol,
3, 4-diméthyl-1,6-bis(2,6,6-triméthyl-1-cyclohexène-1-yl)-1,5-hexadiène-3,4-diol,
3, 4-diméthyl-1,6-bis(2,6,6-triméthyl-2-cyclohexène-1-yl)-1,5-hexadiène-3,4-diol,
1,2-bis(4-hydroxy-3-méthoxyphényl)-1,2-éthanediol,
1,2-bis(1,3-benzodioxol-5-yl)-1,2-éthanediol,
1,2-bis (4-méthoxyphényl)-1,2-éthanediol,
1,2-diphényl-1,2-éthanediol,
1,6-bis (4-méthoxyphényl)-1,5-hexadiène-3, 4-diol ou le 2, 5-dihexyl-1,6-diphényl-1,5-hexadiène-3,4-diol.

12. Procédé pour aromatiser une composition à fumer, par exemple du tabac, ou un aliment, dans lequel on ajoute à la composition à fumer, par exemple du tabac, ou l'on traite un aliment, avec une quantité organoleptiquement efficace d'un 1,2-diol de formule 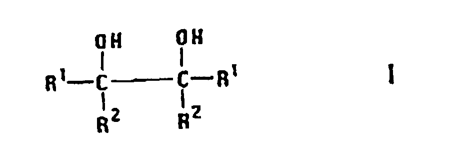
dans laquelle R¹ est l'un des radicaux 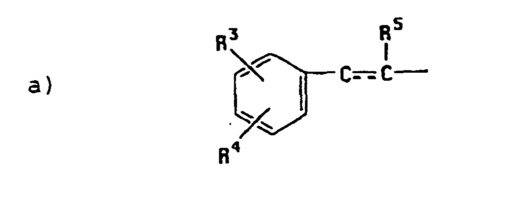 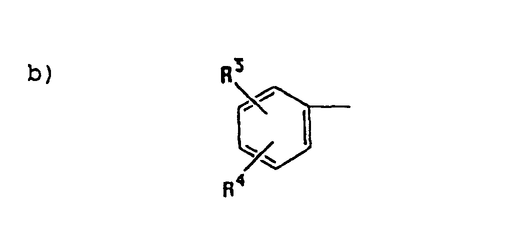 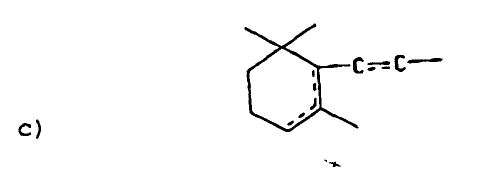 où
R² représente H ou un alkyle inférieur,
R³ et R⁴ représentent chacun H, un alkyle inférieur, un alcoxy inférieur, un hydroxy, ou
R³ et R⁴ ensemble représentent un méthylènedioxy,
R⁵ est un alkyle inférieur,
et les lignes pointillées représentent dans le noyau une liaison supplémentaire et dans la chaîne latérale une liaison facultative.

13. Procédé d'aromatisation d'une composition à fumer, par exemple du tabac, ou d'un aliment, dans lequel on allume et on fait brûler la composition à fumer, par exemple du tabac, ou l'on cuit au four ou fait passer au four à micro-ondes l'aliment contenant une quantité organoleptiquement efficace d'un 1,2-diol de formule 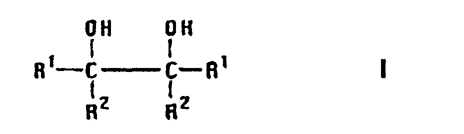
dans laquelle R¹ est l'un des radicaux 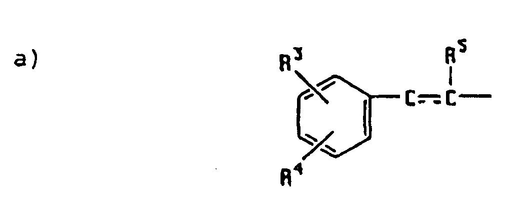 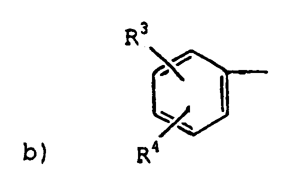 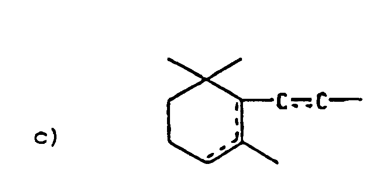 où
R² représente H ou un alkyle inférieur,
R³ et R⁴ représentent chacun H, un alkyle inférieur, un alcoxy inférieur, un hydroxy, ou
R³ et R⁴ ensemble représentent un méthylènedioxy,
R⁵ est un alkyle inférieur,
et les lignes pointillées représentent dans le noyau une liaison supplémentaire et dans la chaîne latérale une liaison facultative.

14. Procédé selon la revendication 12 ou 13, dans lequel le 1,2-diol de formule I est le
1,6-diphényl-1,5-hexadiène-3,4-diol,
3,4-diméthyl-1,6-bis(2,6,6-triméthyl-l-cyclohexène-1-yl) -1,5-hexadiène-3,4-diol,
3,4-diméthyl-1,6-bis(2,6,6-triméthyl-2-cyclohexène-1-yl)-1,5-hexadiène-3,4-diol,
1,2-bis(4-hydroxy-3-méthoxyphényl)-1,2-éthanediol,
1,2-bis(1,3-benzodioxol-5-yl)-1, 2-éthanediol,
1,2-bis(4-méthoxyphényl)-1,2-éthanediol,
1,2-diphényl-1,2-éthanediol,
1,6-bis(4-méthoxyphényl)-1,5-hexadiène-3,4-diol ou le 2,5-dihexyl-1,6-diphényl-1,5-hexadiène-3,4-diol.

15. Le 1,6-bis-(4-méthoxyphényl)-1,5-hexadiène-3,4-diol.

16. Le 2,5-dihexyl-1,6-diphényl-1,5-hexadiène-3,4-diol.

17. Procédé de préparation de 1,6-bis(4-méthoxyphényl)1,5-hexadiène-3,4-diol ou de 2,5-dihexyl-1,6-diphényl-1,5-hexadiène-3,4-diol, dans lequel on soumet le 1,4-méthoxycinnamaldéhyde ou l'a-hexyl-cinnamaldéhyde à une pinacolisation de façon connue.

18. Utilisation des 1,2-diols de formule 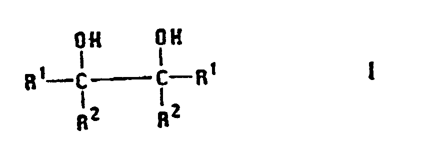
dans laquelle R¹ est l'un des radicaux 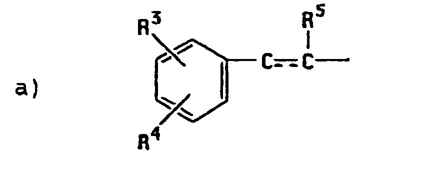 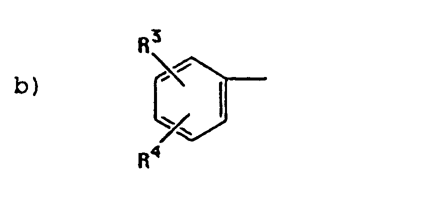 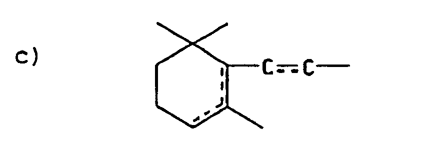 où
R² représente H ou un alkyle inférieur,
R³ et R⁴ représentent chacun H, un alkyle inférieur, un alcoxy inférieur, un hydroxy, ou
R³ et R⁴ ensemble représentent un méthylènedioxy,
R⁵ est un alkyle inférieur,
et les lignes pointillées représentent dans le noyau une liaison supplémentaire et dans la chaîne latérale une liaison facultative,
comme additifs libérant un arôme de façon prolongée pour des compositions à fumer et pour des aliments.
